# FASCICULE DE BREVET EUROPEEN

(11) **EP 3 755 202 B1**
(45) Date de publication et mention de la délivrance du brevet: **20.10.2021**
(21) Numéro de dépôt: 19706277.1
(22) Date de dépôt: 21.02.2019
(51) Int. Cl.: A61B 3/04, A61B 3/103, A61B 3/00, G02C 7/02

(54) **PROCEDE DE COMPARAISON DE VERRES OPTIQUES, MODULE DE COMMANDE ET SYSTEME ASSOCIES**
VERFAHREN ZUM VERGLEICH OPTISCHER LINSEN, ZUGEHÖRIGES STEUERMODUL UND SYSTEM
METHOD FOR COMPARING OPTICAL LENSES, ASSOCIATED CONTROL MODULE AND SYSTEM

(30) Priorité: 23.02.2018 FR 1851617
(43) Date de publication de la demande: 30.12.2020
(73) Titulaire: SIVIEW, 91460 Marcoussis (FR)
(72) Inventeur: GUILLEMAIN, Nathalie, 95320 Saint Leu la Foret (FR); PICHEREAU, Laure, 91460 Marcoussis (FR)
(74) Mandataire: Cabinet Camus Lebkiri
(86) Numéro de dépôt international: PCT/EP2019/054344
(87) Numéro de publication internationale: WO 2019/162394

(56) Documents cités:
- EP-A2- 1 138 253
- WO-A1-2016/142363
- WO-A1-2017/019771
- US-A1- 2006 050 238

## Description

### DOMAINE TECHNIQUE DE L'INVENTION

Le domaine de l'invention est celui des examens de vue qui, pratiqués sur un sujet, permettent de déterminer s'il a besoin d'une correction et laquelle.

L'invention se rapporte plus particulièrement à un procédé de comparaison de verres optiques au moyen d'une tête de réfracteur automatique commandée par un module de commande, par exemple lors d'un examen de vue ou lors d'une simple vérification de la correction visuelle portée. L'invention concerne également un module de commande d'une tête de réfracteur automatique pour la mise en œuvre du procédé de comparaison de verres optiques ainsi qu'un système comportant le module de commande et un écran de sélection pour la mise en œuvre du procédé de comparaison de verres optiques.

### ETAT DE LA TECHNIQUE ANTERIEURE

Un examen de vue d'un sujet débute par une mesure de réfraction objective et se poursuit par une mesure de réfraction subjective.

La mesure de réfraction objective est généralement réalisée par un opérateur au moyen d'un autoréfractomètre. Alternativement à l'utilisation d'un autoréfractomètre, une technique manuelle de skiascopie est parfois utilisée. A défaut l'opérateur peut partir de la précédente correction du sujet examiné.

Lors de la mesure de réfraction subjective, l'opérateur fait tester différentes corrections visuelles au sujet examiné au moyen d'un matériel adapté qui peut être :
- une paire de lunettes d'essai avec des verres d'essai,
- une tête de réfracteur manuelle ou
- une tête de réfracteur automatique.

Dans le premier cas, l'opérateur change manuellement les verres d'essai de la paire de lunettes d'essai. Avec une tête de réfracteur, l'opérateur fait défiler différents verres optiques devant les yeux du sujet examiné, au moyen de mollettes dans le cas d'une tête de réfracteur manuelle ou via une console de commande dans le cas d'une tête de réfracteur automatique. Le document WO 2016/142363 A1, par exemple, qui peut être considéré comme représentant l'état de la technique le plus proche, divulgue un procédé standard de comparaison de verres optiques qui sont placés dans une tête de réfracteur automatique. Les verres sont sélectivement réglables pour ajuster leur puissance sphérique.

Au cours d'une telle mesure de réfraction subjective ou lors d'une vérification de la correction visuelle portée, lorsque l'opérateur a besoin de faire comparer deux verres optiques au sujet examiné, il commence par agencer un premier verre optique devant un œil du sujet examiné pendant que ce dernier observe un écran de lecture sur lequel un ou plusieurs symboles de test sont affichés, puis il remplace le premier verre optique par un deuxième verre optique pendant que le sujet examiné observe toujours le même écran de lecture, avant éventuellement de revenir au premier verre optique, etc. Après un ou plusieurs essais de chacun des deux verres optiques, le sujet examiné dit s'il préfère un verre optique et lequel en fonction de ce qu'il a observé sur l'écran de lecture avec chacun des deux verres optiques. L'opérateur poursuit ensuite la mesure de réfraction subjective ou la vérification de la correction portée sur la base du verre préféré indiqué par le sujet examiné ou en tenant compte du fait que les deux verres sont équivalents du point de vue du sujet examiné.

Il existe toutefois un risque d'erreur par l'opérateur dans la sélection du verre préféré du sujet examiné, en cas de mauvaise compréhension entre le sujet examiné et l'opérateur. Par exemple, une confusion peut naître à force de passer rapidement d'un verre optique à l'autre ou bien si l'opérateur change de verre optique au moment précis où le sujet examiné prend la parole. S'il a un doute, l'opérateur peut vérifier sa compréhension auprès du sujet examiné. En revanche, le sujet examiné ne peut pas vérifier que la sélection de l'opérateur est correcte. Dans le même temps, une telle comparaison n'est généralement qu'une étape d'un examen de vue ou d'une vérification de correction visuelle portée et il est souhaité de pouvoir aller vite. Par ailleurs, en cas d'erreur, l'examen de vue ou la vérification de correction visuelle portée risque de ne pas aboutir ou de mettre plus de temps à aboutir ou d'aboutir à un résultat erroné.

Il est donc recherché une solution permettant, notamment dans le cadre d'un examen de vue ou d'une vérification de correction visuelle portée, de comparer deux verres optiques rapidement et sans se tromper sur le verre optique retenu.

### EXPOSE DE L'INVENTION

Dans ce contexte, l'invention vise à remédier à tout ou partie des inconvénients de l'état de la technique identifiés ci-dessus.

A cette fin, un premier aspect de l'invention concerne un procédé de comparaison de premier et second verres optiques au moyen d'une tête de réfracteur automatique commandée par un module de commande, le procédé comportant les étapes suivantes selon lesquelles le module de commande :
- affiche sur un écran de sélection une proposition d'un premier verre optique et une proposition d'un second verre optique ;
- reçoit une sélection de la proposition du premier verre optique ;
- agence le premier verre optique dans un emplacement de la tête de réfracteur automatique à réception de la sélection de la proposition du premier verre optique ;
- reçoit une sélection de la proposition du second verre optique ;
- agence le second verre optique dans l'emplacement de la tête de réfracteur automatique à réception de la sélection de la proposition du second verre optique ;
- affiche sur l'écran de sélection une pluralité de symboles de validation indiquant chacun un résultat possible de la comparaison ;
le procédé étant tel que :
- lorsque le module de commande reçoit la sélection de la proposition du premier verre optique pour la première fois, la proposition du second verre optique est sélectionnable sur l'écran de sélection et aucun symbole de validation n'est sélectionnable sur l'écran de sélection ;
- lorsque le module de commande reçoit la sélection de la proposition du second verre optique, la proposition du premier verre optique est sélectionnable sur l'écran de sélection et des premier et deuxième symboles de validation sont sélectionnables sur l'écran de sélection, le premier symbole indiquant que les premier et second verres optiques sont pareils et le deuxième symbole indiquant que le second verre optique est mieux que le premier verre optique ;
- lorsque le module de commande reçoit la sélection de la proposition du premier verre optique pour la deuxième fois ou plus, la proposition du second verre optique est sélectionnable sur l'écran de sélection et le premier symbole et un troisième symbole sont sélectionnables sur l'écran de sélection, le troisième symbole indiquant que le premier verre optique est mieux que le second verre optique.

Un deuxième aspect de l'invention concerne un module de commande d'une tête de réfracteur automatique pour la mise en œuvre d'un procédé de comparaison de premier et second verres optiques selon le premier aspect de l'invention, le module de commande ayant :
- des moyens pour afficher simultanément sur un écran de sélection une proposition d'un premier verre optique, une proposition d'un second verre optique et tout ou partie d'une pluralité de symboles de validation indiquant chacun un résultat possible d'une comparaison entre les premier et second verres optiques ;
- des moyens pour recevoir une sélection de la proposition du premier ou du second verre optique ou d'un symbole de validation ;
- des moyens pour, à réception de la sélection de la proposition du premier ou du second verre optique, agencer ledit premier ou second verre optique dans un emplacement de la tête de réfracteur automatique ;
les moyens d'affichage sur l'écran de sélection et de réception d'une sélection étant tels que :
- lorsque le module de commande reçoit la sélection de la proposition du premier verre optique pour la première fois, la proposition du second verre optique est sélectionnable sur l'écran de sélection et aucun symbole de validation n'est sélectionnable sur l'écran de sélection ;
- lorsque le module de commande reçoit la sélection de la proposition du second verre optique, la proposition du premier verre optique est sélectionnable sur l'écran de sélection et des premier et deuxième symboles de validation sont sélectionnables sur l'écran de sélection, le premier symbole indiquant que les premier et second verres optiques sont pareils et le deuxième symbole indiquant que le second verre optique est mieux que le premier verre optique ;
- lorsque le module de commande reçoit la sélection de la proposition du premier verre optique pour la deuxième fois ou plus, la proposition du second verre est sélectionnable sur l'écran de sélection et le premier symbole et un troisième symbole sont sélectionnables sur l'écran de sélection, le troisième symbole indiquant que le premier verre optique est mieux que le second verre optique.

Un troisième aspect de l'invention concerne un système pour la mise en œuvre d'un procédé de comparaison de premier et second verres optiques selon le premier aspect de l'invention, comportant un module de commande d'une tête de réfracteur automatique selon le deuxième aspect de l'invention ainsi qu'un écran de sélection.

Outre les caractéristiques qui viennent d'être mentionnées dans les paragraphes précédents, le procédé de comparaison de premier et second verres optiques selon le premier aspect de l'invention, le module de commande selon le deuxième aspect de l'invention et le système selon le troisième aspect de l'invention peuvent présenter une ou plusieurs caractéristiques complémentaires parmi les suivantes, considérées individuellement ou selon toutes les combinaisons techniquement possibles :
- Lorsque la deuxième étape a lieu pour la première fois, elle est réalisée automatiquement par le module de commande sans intervention d'un opérateur.
- Lorsque le module de commande reçoit la sélection de la proposition du premier verre optique pour la première fois, le module de commande n'affiche aucun symbole de validation sur l'écran de sélection.
- Lorsque le module de commande reçoit la sélection de la proposition du second verre optique, le module de commande affiche les premier et deuxième symboles de validation mais pas le troisième symbole de validation.

- Lorsque le module de commande reçoit la sélection de la proposition du premier verre optique pour la deuxième fois ou plus, le module de commande affiche les premier et troisième symboles de validation mais pas le deuxième symbole de validation.
- Le système selon le troisième aspect de l'invention comporte également un écran de lecture, l'écran de sélection et l'écran de lecture étant deux écrans distincts ou un seul et même écran.

### BREVE DESCRIPTION DES FIGURES

D'autres caractéristiques et avantages de l'invention ressortiront à la lecture de la description qui suit, en référence aux figures annexées.
- La figure 1 montre un diagramme des étapes du procédé selon le premier aspect de l'invention.
- La figure 2 montre un exemple d'affichage par le module de commande d'un premier verre optique et d'une proposition d'un second verre optique sur un écran de sélection d'une proposition.
- La figure 3a montre un exemple d'affichage par le module de commande de premier et troisième symboles de validations sur l'écran de sélection, lorsque la proposition du premier verre optique est sélectionnée.
- La figure 3b montre un agencement d'un premier verre optique dans un emplacement d'un support, selon une troisième étape du procédé selon le premier aspect de l'invention.
- La figure 4a montre un exemple d'affichage par le module de commande de premier et deuxième symboles de validations sur l'écran de sélection, lorsque la proposition du second verre optique est sélectionnée
- La figure 4b montre un agencement d'un second verre optique dans l'emplacement du support, selon une cinquième étape du procédé selon le premier aspect de l'invention.

Pour plus de clarté, des éléments identiques ou similaires sont repérés par des signes de référence identiques sur l'ensemble des figures.

### DESCRIPTION DETAILLEE D'AU MOINS UN MODE DE REALISATION

Un premier aspect de l'invention concerne un procédé de comparaison de premier et second verres optiques au moyen d'une tête de réfracteur automatique commandée par un module de commande. Un deuxième aspect de l'invention concerne un module de commande d'une tête de réfracteur automatique pour la mise en œuvre du procédé de comparaison de premier et second verres optiques selon le premier aspect de l'invention. Un troisième aspect de l'invention concerne un système comportant le module de commande selon le deuxième aspect de l'invention et un écran de sélection pour la mise en œuvre du procédé selon le premier aspect de l'invention.

### Définitions

Dans le cadre de la présente invention, on entend par « verre optique » un élément optique de qualité adaptée à la réalisation de lunettes de vue, sans préjuger du matériau dans lequel il est réalisé et qui peut notamment être du verre ou du plastique. Les termes « verre ophtalmique » ou « verre correcteur » peuvent également être employés. Le terme « lentille optique » peut également être employé en toute rigueur, à condition de bien l'interpréter au sens d'un élément optique de qualité adaptée à la réalisation de lunettes de vue, par opposition à une lentille cornéenne ou lentille de contact.

Dans le cadre de la présente invention, on entend par « tête de réfracteur » un support comportant des premier et second emplacements, un pour chaque œil, pouvant chacun recevoir un élément qui est soit un cache opaque, soit un verre optique. Dans le cadre de la présente invention, on entend par « verre optique agencé dans un emplacement de la tête de réfracteur » soit un unique verre optique, soit une combinaison de plusieurs verres optiques, permettant de réaliser une certaine correction optique. La tête de réfracteur est destinée à être placée devant le sujet examiné de manière que le sujet examiné regarde, à travers les premier et second emplacements, un ou plusieurs symboles de test affichés sur un écran de lecture afin de les lire ou de les identifier.

On entend par « tête de réfracteur automatique » une tête de réfracteur pour laquelle l'agencement successif de différents éléments dans les premier et second emplacements est réalisé par un opérateur via le module de commande. Une étape d'agencement d'un élément dans un emplacement peut commencer le cas échéant par une étape de retrait d'un précédent élément dudit emplacement.

Dans le cadre du procédé de comparaison de verres optiques selon le premier aspect de l'invention, la tête de réfracteur automatique peut être utilisée en monoculaire œil droit, en monoculaire œil gauche ou en binoculaire. En monoculaire œil droit, un verre optique est agencé dans l'emplacement correspondant à l'œil droit tandis qu'un cache est agencé dans l'emplacement correspondant à l'œil gauche. En monoculaire œil gauche, un verre optique est agencé dans l'emplacement correspondant à l'œil gauche tandis qu'un cache est agencé dans l'emplacement correspondant à l'œil droit. En binoculaire, un verre optique est agencé dans chacun des deux emplacements.

### Procédé selon le premier aspect de l'invention

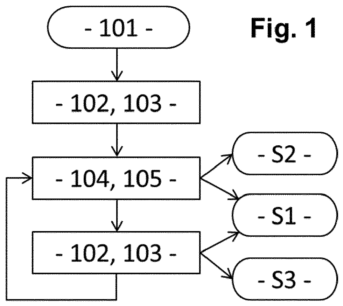

Le procédé 100 selon le premier aspect de l'invention est mis en œuvre au moyen d'une tête de réfracteur automatique T commandée par le module de commande selon le deuxième aspect de l'invention. La figure 1 montre un diagramme des étapes du procédé 100.

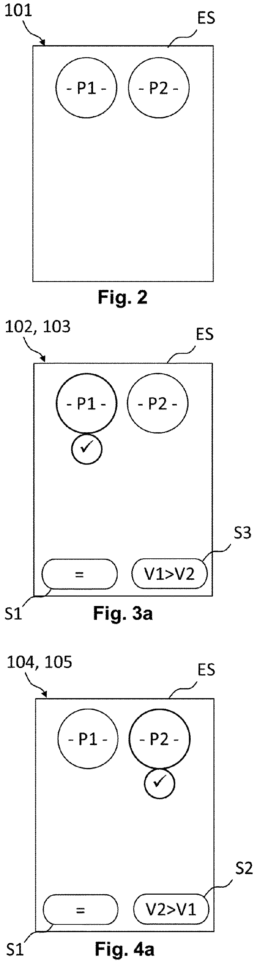

Selon une première étape 101 illustrée à la figure 2, le module de commande affiche sur l'écran de sélection ES une proposition P1 d'un premier verre optique V1 et une proposition P2 d'un second verre optique V2.

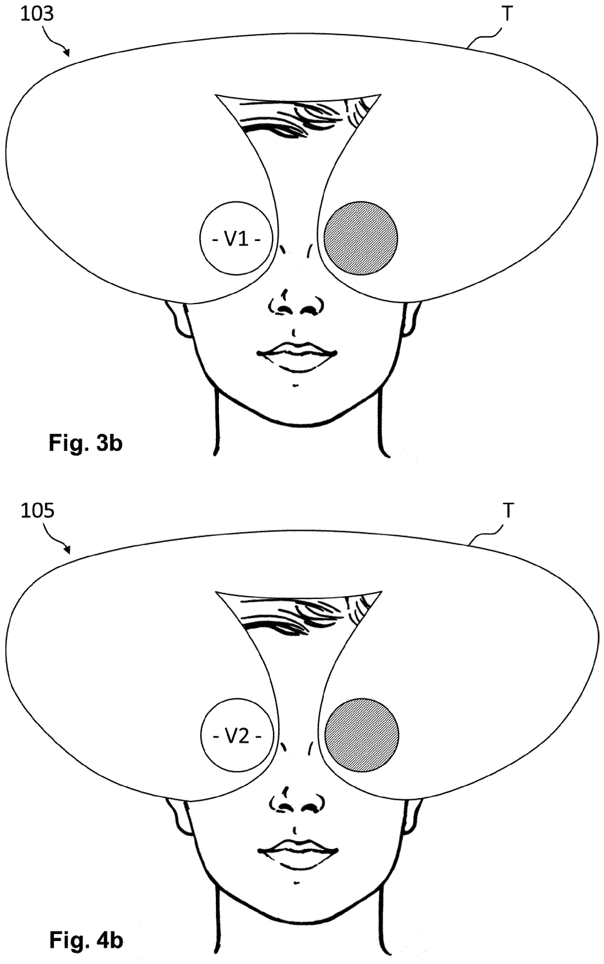

Selon une deuxième étape 102 illustrée à la figure 3a, l'opérateur sélectionne la proposition P1 du premier verre optique V1 et le module de commande reçoit la sélection de la proposition P1 du premier verre optique V1. Selon la deuxième étape 102 lorsqu'elle a lieu pour la première fois, la proposition P1 du premier verre optique V1 est préférentiellement automatiquement sélectionnée, sans intervention de l'opérateur. Selon une troisième étape 103, à réception de la sélection de la proposition P1 du premier verre optique V1, le module de commande agence ledit premier verre optique V1 dans un emplacement de la tête de réfracteur automatique T. Si cet emplacement est déjà occupé par un autre élément, le module de commande commence par retirer l'autre élément pour pouvoir placer le premier verre optique V1. La figure 3b illustre l'agencement selon la troisième étape 103.

Selon la troisième étape 103 lorsqu'elle a lieu pour la première fois, à réception de la sélection de la proposition P1 du premier verre optique V1, le module de commande affiche en outre préférentiellement sur l'écran de sélection ES des premier et troisième symboles de validation S1, S3, le premier symbole S1 indiquant que les premier et second verres V1, V2 sont pareils, le troisième symbole S3 indiquant que le premier verre optique V1 est mieux que le second verre optique V2, sans toutefois que les premier et troisième symboles de validation S1, S3 affichés ne soient sélectionnables. La figure 3a illustre cet affichage préférentiel. Un deuxième symbole de validation S2 indiquant que le second verre optique V2 est mieux que le premier verre optique V1 pourrait être affiché en plus tout en n'étant pas sélectionnable non plus. Alternativement, selon la troisième étape 103 lorsqu'elle a lieu pour la première fois, à réception de la sélection de la proposition P1 du premier verre optique V1, le module de commande n'affiche aucun symbole de validation sur l'écran de sélection ES.

Selon une quatrième étape 104 illustrée à la figure 4a, le module de commande reçoit une sélection de la proposition P2 du second verre optique V2. Selon une cinquième étape 105, à réception de la sélection de la proposition P2 du second verre optique V2, le module de commande agence ledit second verre optique V2 dans l'emplacement de la tête de réfracteur automatique T - en commençant par retirer le premier verre optique V1. La figure 4a illustre l'agencement selon la cinquième étape 105.

Selon la cinquième étape 105, à réception de la sélection de la proposition P2 du second verre optique V2, le module de commande affiche en outre sur l'écran de sélection ES le premier symbole de validation S1 et un deuxième symbole de validation S2 indiquant que le second verre optique V2 est mieux que le premier verre optique V1, chacun des premier et deuxième symboles S1, S2 étant sélectionnable. En revanche, le troisième symbole de validation S3, qui permet d'indiquer que le premier verre V1 est mieux que le second verre V2, n'est pas sélectionnable et de manière préférentielle il n'est pas affiché du tout. La figure 4a illustre cet affichage préférentiel.

Après la cinquième étape 105, le module de commande peut :
- soit recevoir une sélection du premier symbole de validation S1, ce qui termine le procédé de comparaison 100 en indiquant que les premier et second verres optiques V1, V2 sont pareils ;
- soit recevoir une sélection du deuxième symbole de validation S2, ce qui termine le procédé de comparaison 100 en indiquant que le second verre optique V2 est mieux que le premier verre optique V1 ;
- soit recevoir à nouveau la sélection de la proposition P1 du premier verre optique V1 selon la deuxième étape 102, et agencer le premier verre optique V1 dans l'emplacement de la tête de réfracteur automatique selon la troisième étape 103 - en commençant cette fois par retirer le second verre optique V2.

Selon la troisième étape 103 lorsqu'elle a lieu pour la deuxième fois ou plus, à réception de la sélection de la proposition P1 du premier verre optique V1, le module de commande affiche en outre sur l'écran de sélection ES le premier symbole de validation S1 et un troisième symbole de validation S3 indiquant que le premier verre optique V1 est mieux que le second verre optique V2, chacun des premier et troisième symboles S1, S3 étant sélectionnable. En revanche, le deuxième symbole de validation S2, qui permet d'indiquer que le second verre optique V2 est mieux que le premier verre optique V1, n'est pas sélectionnable et de manière préférentielle il n'est pas affiché du tout. La figure 3a illustre cet affichage préférentiel.

Après la troisième étape 103 lorsqu'elle a lieu pour la deuxième fois ou plus, le module de commande peut :
- soit recevoir une sélection du premier symbole de validation S1 indiquant que les premier et second verres optiques V1, V2 sont pareils ;
- soit recevoir une sélection du troisième symbole de validation S3 indiquant que le premier verre optique V1 est mieux que le second verre optique V2,
- soit recevoir à nouveau la sélection de la proposition P2 du second verre optique V2 selon la quatrième étape 104, et agencer le second verre optique V2 dans l'emplacement de la tête de réfracteur automatique selon la cinquième étape 105 - en commençant par retirer le premier verre optique V1.

Les figures 3b et 4b montrent un exemple selon lequel la tête de réfracteur automatique est utilisée en monoculaire œil droit : les premier et second verres optiques V1, V2 sont successivement agencés dans l'emplacement correspondant à l'œil droit tandis que l'emplacement correspondant à l'œil gauche est occupé par un cache opaque. L'invention s'applique naturellement également aux cas monoculaire œil gauche, en agençant successivement les premier et second verres optiques V1, V2 dans l'emplacement correspondant à l'œil gauche tandis que l'emplacement correspondant à l'œil droit est occupé par un cache opaque ; et binoculaire, en testant un premier ensemble d'un premier verre optique pour l'œil droit et d'un premier verre optique pour l'œil gauche, puis un second ensemble d'un second verre optique pour l'œil droit et d'un second verre optique pour l'œil gauche. Dans le cas binoculaire, le premier verre optique pour l'œil droit et le premier verre optique pour l'œil gauche peuvent être identiques ou différents ; de même le second verre optique pour l'œil droit et le second verre optique pour l'œil gauche peuvent être identiques ou différents.

### Module de commande selon le deuxième aspect de l'invention

Le module de commande selon le deuxième aspect de l'invention comporte quant à lui des moyens pour afficher simultanément sur l'écran de sélection ES la proposition P1 du premier verre optique V1, la proposition P2 du second verre optique V2 et tout ou partie des symboles de validation S1, S2, S3 indiquant chacun un résultat possible d'une comparaison entre les premier et second verres optiques V1, V2, préférentiellement soit les premier et deuxième symboles S1, S2, soit les premier et troisième symboles S1, S3.

Le module de commande selon le deuxième aspect de l'invention comporte également des moyens pour recevoir une sélection de la proposition P1, P2 du premier ou du second verre optique V1, V2 ou d'un symbole de validation S1, S2, S3. Le module de commande ne peut recevoir une sélection d'une proposition P1, P2 ou d'un symbole S1, S2, S3 que si ladite proposition ou ledit symbole est affiché au préalable. Toute proposition affichée est sélectionnable mais tout symbole affiché n'est pas nécessairement sélectionnable.

Enfin, le module de commande selon le deuxième aspect de l'invention comporte des moyens pour, à réception de la sélection de la proposition P1, P2 du premier ou du second verre optique V1, V2, agencer ledit premier ou second verre optique V1, V2 dans un emplacement de la tête de réfracteur automatique T.

De manière préférentielle, le module de commande selon le deuxième aspect de l'invention comporte des moyens pour afficher un symbole de test ou une pluralité de symboles de test sur l'écran de lecture. Le module de commande selon le deuxième aspect de l'invention peut également comporter des moyens pour dupliquer sur l'écran de lecture tout ou partie de l'affichage réalisé sur l'écran de sélection et/ou pour dupliquer sur l'écran de sélection tout ou partie de l'affichage réalisé sur l'écran de lecture.

Par ailleurs, les moyens d'affichage sur l'écran de sélection ES et de réception d'une sélection sont tels que :
- lorsque le module de commande reçoit la sélection de la proposition P1 du premier verre optique V1 pour la première fois, la proposition P2 du second verre optique V2 est sélectionnable sur l'écran de sélection ES et aucun symbole de validation S1, S2, S3 n'est sélectionnable sur l'écran de sélection ES ;
- lorsque le module de commande reçoit la sélection de la proposition P2 du second verre optique V2, la proposition P1 du premier verre optique V1 est sélectionnable sur l'écran de sélection ES et les premier et deuxième symboles de validation S1, S2 sont sélectionnables sur l'écran de sélection ES.
- lorsque le module de commande reçoit la sélection de la proposition P1 du premier verre optique V1 pour la deuxième fois ou plus, la proposition P2 du second verre V2 est sélectionnable sur l'écran de sélection ES et les premier et troisième symboles S1, S3 sont sélectionnables sur l'écran de sélection ES.

### Ecran de sélection et écran de lecture

L'opérateur interagit avec l'écran de sélection ES pour sélectionner une proposition P1, P2 ou un symbole S1, S2, S3 affichés. Le sujet examiné interagit quant à lui avec l'écran de lecture pour lire ou identifier, à travers la tête de réfracteur automatique T, un ou plusieurs symboles de test affichés.

L'écran de sélection ES et l'écran de lecture sont préférentiellement deux écrans distincts, par exemple une tablette pour l'écran de sélection ES et un écran de projection pour l'écran de lecture. Dans ce cas, l'affichage réalisé sur l'écran de sélection ES et sur l'écran de lecture peut être identique ou différencié.

Alternativement, l'écran de sélection ES et l'écran de lecture peuvent être un seul et même écran, par exemple un écran de projection, donc avec un seul et unique affichage.

### Opérateur et sujet examiné

On définit l'opérateur comme étant celui qui interagit avec l'écran de sélection ES et le module de commande. L'opérateur est typiquement un praticien, tel qu'un ophtalmologue, un opticien ou même, dans le cadre de la présente invention, un assistant sans compétences particulières en physique de la réfraction et physiologie du système visuel. On définit le sujet examiné comme étant celui qui regarde l'écran de lecture à travers la tête de réfracteur automatique T. Le sujet examiné est typiquement un patient ou client du praticien.

Le sujet examiné peut lui-même être opérateur. Le sujet examiné peut être l'unique opérateur en l'absence de tout praticien. Alternativement, deux opérateurs distincts, par exemple le sujet examiné et un praticien, peuvent se relayer. Lorsque le sujet examiné est opérateur, soit l'écran de lecture forme un seul et même écran avec l'écran de sélection, soit l'écran de lecture est un deuxième écran de sélection.

### Marqueurs sur l'écran de lecture

Préférentiellement, le module de commande comporte des moyens pour afficher sur l'écran de lecture :
- un premier marqueur propre à la proposition P1 du premier verre optique V1 à réception de la sélection de la proposition P1 du premier verre optique V1, et
- un second marqueur propre à la proposition P2 du second verre optique V2 à réception de la sélection de la proposition P2 du second verre optique V2.

Les premier et second marqueurs permettent au sujet examiné de savoir quelle proposition de verre il est en train de tester.

Les premier et second marqueurs présentent une taille qui est ajustée afin que leur angle de discrimination soit suffisamment grand pour être perçu par le sujet examiné même s'il ne parvient pas par ailleurs à lire ou identifier les symboles de test. Les premier et second marqueurs sont préférentiellement affichés dans des première et deuxième zones distinctes de l'écran de lecture.

### Système selon le troisième aspect de l'invention

Le système selon le troisième aspect de l'invention comporte le module de commande selon le deuxième aspect de l'invention ainsi que l'écran de sélection ES.

L'écran de sélection ES peut être intégré avec le module de commande. C'est par exemple le cas d'un module de commande sous forme de tablette tactile ou d'ordinateur portable. Alternativement, l'écran de sélection ES peut être distinct du module de commande. C'est par exemple le cas d'un module de commande connecté avec un écran distant de type écran d'ordinateur, écran de télévision ou écran de projection.

Le système selon le troisième aspect de l'invention peut également comporter l'écran de lecture. Dans ce cas, l'écran de sélection ES et l'écran de lecture peuvent être un seul et même écran ou deux écrans distincts.

### Etapes et moyens de sélection

Chaque étape de sélection, soit d'une proposition P1, P2 d'un verre, soit d'un symbole de validation S1, S2, S3, est réalisée par l'opérateur réalisant une action de sélection en interaction avec l'écran de sélection ES. L'action de sélection peut être réalisée de différentes manières, dont plusieurs exemples non limitatifs sont listés ci-après.

Si l'action de sélection est réalisée sur une proposition P1, P2 ou un symbole de validation S1, S2, S3 sélectionnable, elle entraîne la transmission d'une sélection au module de commande, qui la prend en compte en réalisant les étapes d'affichage et/ou d'agencement telles que précédemment décrites. Si l'action de sélection est réalisée sur une proposition P1, P2 ou un symbole de validation S1, S2, S3 qui n'est pas sélectionnable, elle n'entraîne aucune transmission de sélection au module de commande.

En outre, si l'action de sélection est réalisée sur une proposition P1, P2 ou un symbole de validation S1, S2, S3 sélectionnable, elle entraîne préférentiellement une modification graphique de l'aspect de l'affichage de manière à informer l'opérateur que son action de sélection a fonctionné, tandis que si l'action de sélection est réalisée sur une proposition P1, P2 ou un symbole de validation S1, S2, S3 non sélectionnable, elle n'entraîne aucune modification graphique de l'aspect de l'affichage de manière à informer l'opérateur que son action de sélection n'a pas fonctionné.

L'action de sélection est préférentiellement réalisée au moyen d'un écran de sélection ES tactile : l'opérateur touche une zone de l'écran sur laquelle se trouve une proposition P1, P2 d'un verre ou un symbole de validation S1, S2, S3 afin de sélectionner ladite proposition ou ledit symbole de validation.

Alternativement, l'action de sélection peut être réalisée au moyen d'une interface qui est par exemple
- de type souris : l'opérateur positionne le curseur de la souris sur la zone de l'écran sur laquelle se trouve la proposition P1, P2 d'un verre ou le symbole de validation S1, S2, S3 et clique sur cette zone afin de sélectionner ladite proposition ou ledit symbole de validation ; ou
- de type clavier : chaque proposition P1, P2 d'un verre est identifiée par une touche ou combinaison de touches qui lui est propre, par exemple la touche « 1 » pour la proposition P1 du premier verre et la touche « 2 » pour la proposition P2 du second verre ; de même, le premier symbole de validation S1 (celui qui indique que les premier et second verres optiques sont pareils) est identifié par une touche ou combinaison de touches qui lui est propre, par exemple la touche « espace ». Comme les deuxième et troisième symboles de validation S2, S3 ne sont pas simultanément sélectionnables, ils sont quant à eux avantageusement identifiés tous les deux par une seule et même touche ou combinaison de touches qui leur est propre, par exemple la touche « entrée ». L'opérateur appuie sur une touche ou combinaison de touches afin de sélectionner la proposition ou le symbole de validation correspondant.
- de type manette directionnelle ou joystick : chaque proposition P1, P2 d'un verre est identifiée par une direction qui lui est propre, par exemple vers la gauche pour la proposition P1 du premier verre et vers la droite pour la proposition P2 du second verre, les directions correspondant préférentiellement, le cas échéant, aux première et deuxième zones distinctes de l'écran de lecture sur lesquelles sont affichés les premier et second marqueurs. La manette directionnelle comporte également un premier bouton permettant de sélectionner le premier symbole S1 et un second bouton permettant de sélectionner le deuxième symbole S2 ou le troisième symbole S3. L'opérateur oriente la manette directionnelle dans une direction donnée afin de sélectionner la proposition correspondante, ou appuie sur un bouton afin de sélectionner le symbole de validation correspondant.

Selon une autre alternative, la sélection est réalisée au moyen d'une commande vocale, via un équipement de type microphone et un module de traitement du signal adapté dans le module de commande : chaque proposition P1, P2 d'un verre est identifiée par un mot ou groupe de mots qui lui est propre, par exemple le mot « un » pour la proposition P1 du premier verre et le mot « deux » pour la proposition P2 du second verre ; de même, le premier symbole de validation S1 (celui qui indique que les premier et second verres optiques sont pareils) est identifié par un mot ou groupe de mots qui lui est propre, par exemple le mot « pareil » ou « égal ». Comme les deuxième et troisième symboles de validation S2, S3 ne sont pas simultanément sélectionnables, ils sont quant à eux avantageusement identifiés tous les deux par un seul et même mot ou groupe de mots qui leur est propre, par exemple le mot « mieux » ou « valider ». L'opérateur prononce un mot ou groupe de mots afin de sélectionner la proposition ou le symbole de validation correspondant.

Selon encore une autre alternative, la sélection est réalisée au moyen d'une commande gestuelle, via un équipement de type caméra et un module de traitement du signal adapté dans le module de commande. Selon une première variante, chaque proposition P1, P2 d'un verre est identifiée par une position ou un mouvement de la main qui lui est propre, par exemple l'index levé pour la proposition P1 du premier verre et l'index et le majeur levés pour la proposition P2 du second verre ; de même, le premier symbole de validation S1 (celui qui indique que les premier et second verres optiques sont pareils) est identifié par une position ou un mouvement de la main qui lui est propre, par exemple le poing fermé. Comme les deuxième et troisième symboles de validation S2, S3 ne sont pas simultanément sélectionnables, ils sont quant à eux avantageusement identifiés tous les deux par une seule et même position ou mouvement de la main qui leur est propre, par exemple le pouce levé. Selon une deuxième variante, l'opérateur utilise un doigt, par exemple son index, comme un pointeur afin de désigner la zone de l'écran sur laquelle se trouve la proposition ou le symbole de validation qu'il souhaite sélectionner. Dans chacune des deux variantes, l'opérateur positionne sa main ou réalise un mouvement afin de sélectionner la proposition ou le symbole de validation correspondant.

Les différentes actions de sélection du procédé 100 selon le premier aspect de l'invention peuvent être toutes réalisées d'une seule et même manière, par exemple au moyen d'un écran de sélection tactile. Alternativement, une combinaison de plusieurs techniques différentes peut être utilisée pour réaliser les différentes actions de sélection du procédé 100, par exemple les actions de sélection d'une proposition via un écran de sélection tactile ou via une commande gestuelle, et les actions de sélection d'un symbole de validation via une commande vocale, etc.

## Revendications

1. Procédé (100) de comparaison de premier et second verres optiques (V1, V2) au moyen d'une tête de réfracteur automatique (T) commandée par un module de commande, le procédé (100) comportant les étapes suivantes selon lesquelles le module de commande :
- affiche (101) sur un écran de sélection (ES) une proposition (P1) d'un premier verre optique (V1) et une proposition (P2) d'un second verre optique (V2) ;
- reçoit (102) une sélection de la proposition (P1) du premier verre optique (V1) ;
- agence (103) le premier verre optique (V1) dans un emplacement de la tête de réfracteur automatique (T) à réception de la sélection de la proposition (P1) du premier verre optique (V1) ;
- reçoit (104) une sélection de la proposition (P2) du second verre optique (V2) ;
- agence (105) le second verre optique (V2) dans l'emplacement de la tête de réfracteur automatique (T) à réception de la sélection de la proposition (P2) du second verre optique (V2) ;
- affiche (103, 105) sur l'écran de sélection (ES) une pluralité de symboles de validation (S1, S2, S3) indiquant chacun un résultat possible de la comparaison ;
le procédé (100) étant tel que :
- lorsque le module de commande reçoit la sélection de la proposition (P1) du premier verre optique (V1) pour la première fois, la proposition (P2) du second verre optique (V2) est sélectionnable sur l'écran de sélection (ES) et aucun symbole de validation (S1, S2, S3) n'est sélectionnable sur l'écran de sélection (ES) ;
- lorsque le module de commande reçoit la sélection de la proposition (P2) du second verre optique (V2), la proposition (P1) du premier verre optique (V1) est sélectionnable sur l'écran de sélection (ES) et des premier et deuxième symboles de validation (S1, S2) sont sélectionnables sur l'écran de sélection (ES), le premier symbole (S1) indiquant que les premier et second verres optiques (V1, V2) sont pareils et le deuxième symbole (S2) indiquant que le second verre optique (V2) est mieux que le premier verre optique (V1) ;
- lorsque le module de commande reçoit la sélection de la proposition (P1) du premier verre optique (V1) pour la deuxième fois ou plus, la proposition (P2) du second verre optique (V2) est sélectionnable sur l'écran de sélection (ES) et le premier symbole (S1) et un troisième symbole (S3) sont sélectionnables sur l'écran de sélection (ES), le troisième symbole (S3) indiquant que le premier verre optique (V1) est mieux que le second verre optique (V2).

2. Procédé (100) selon la revendication précédente **caractérisé en ce que** lorsque la deuxième étape (102) a lieu pour la première fois, elle est réalisée automatiquement par le module de commande sans intervention d'un opérateur.

3. Procédé (100) selon l'une quelconque des revendications précédentes **caractérisé en ce que** lorsque le module de commande reçoit la sélection de la proposition (P1) du premier verre optique (V1) pour la première fois, le module de commande n'affiche aucun symbole de validation (S1, S2, S3) sur l'écran de sélection (ES).

4. Procédé (100) selon l'une quelconque des revendications précédentes **caractérisé en ce que** lorsque le module de commande reçoit la sélection de la proposition (P2) du second verre optique (V2), le module de commande affiche les premier et deuxième symboles de validation (S1, S2) mais pas le troisième symbole de validation (S3).

5. Procédé (100) selon l'une quelconque des revendications précédentes **caractérisé en ce que** lorsque le module de commande reçoit la sélection de la proposition (P1) du premier verre optique (V1) pour la deuxième fois ou plus, le module de commande affiche les premier et troisième symboles de validation (S1, S3) mais pas le deuxième symbole de validation (S2).

6. Module de commande d'une tête de réfracteur automatique (T) pour la mise en œuvre d'un procédé (100) de comparaison de premier et second verres optiques selon l'une quelconque des revendications précédentes, le module de commande ayant :
- des moyens pour afficher simultanément sur un écran de sélection (ES) une proposition (P1) d'un premier verre optique (V1), une proposition (P2) d'un second verre optique (V2) et tout ou partie d'une pluralité de symboles de validation (S1, S2, S3) indiquant chacun un résultat possible d'une comparaison entre les premier et second verres optiques (V1, V2) ;
- des moyens pour recevoir une sélection de la proposition (P1, P2) du premier ou du second verre optique (V1, V2) ou d'un symbole de validation (S1, S2, S3) ;
- des moyens pour, à réception de la sélection de la proposition (P1, P2) du premier ou du second verre optique (V1, V2), agencer ledit premier ou second verre optique (V1, V2) dans un emplacement de la tête de réfracteur automatique (T) ;
les moyens d'affichage sur l'écran de sélection et de réception d'une sélection étant tels que :
- lorsque le module de commande reçoit la sélection de la proposition (P1) du premier verre optique (V1) pour la première fois, la proposition (P2) du second verre optique (V2) est sélectionnable sur l'écran de sélection (ES) et aucun symbole de validation (S1, S2, S3) n'est sélectionnable sur l'écran de sélection (ES) ;
- lorsque le module de commande reçoit la sélection de la proposition (P2) du second verre optique (V2), la proposition (P1) du premier verre optique (V1) est sélectionnable sur l'écran de sélection (ES) et des premier et deuxième symboles de validation (S1, S2) sont sélectionnables sur l'écran de sélection (ES), le premier symbole (S1) indiquant que les premier et second verres optiques (V1, V2) sont pareils et le deuxième symbole (S2) indiquant que le second verre optique (V2) est mieux que le premier verre optique (V1) ;
- lorsque le module de commande reçoit la sélection de la proposition (P1) du premier verre optique (V1) pour la deuxième fois ou plus, la proposition (P2) du second verre (V2) est sélectionnable sur l'écran de sélection (ES) et le premier symbole (S1) et un troisième symbole (S3) sont sélectionnables sur l'écran de sélection (ES), le troisième symbole (S3) indiquant que le premier verre optique (V1) est mieux que le second verre optique (V2).

7. Système pour la mise en œuvre d'un procédé (100) de comparaison de premier et second verres optiques selon l'une quelconque des revendications 1 à 5, comportant le module de commande d'une tête de réfracteur automatique (T) selon la revendication 6 ainsi que l'écran de sélection (ES).

8. Système selon la revendication précédente **caractérisé en ce qu'**il comporte en outre un écran de lecture.

9. Système selon la revendication précédente **caractérisé en ce que** l'écran de sélection (ES) et l'écran de lecture sont deux écrans distincts.

10. Système selon la revendication 8 **caractérisé en ce que** l'écran de sélection (ES) et l'écran de lecture sont un seul et même écran.

## Patentansprüche

1. Vergleichsverfahren (100) eines ersten und eines zweiten optischen Glases (V1, V2) mittels eines automatischen Rückstrahlerkopfes (T), der durch ein Steuermodul gesteuert wird, wobei das Verfahren (100) die folgenden Schritte umfasst, nach denen das Steuermodul:
- auf einem Auswahlbildschirm (ES) einen Vorschlag (P1) eines ersten optischen Glases (V1) und einen Vorschlag (P2) eines zweiten optischen Glases (V2) anzeigt (101);
- eine Auswahl des Vorschlags (P1) des ersten optischen Glases (V1) empfängt (102);
- das erste optische Glas (V1) beim Empfang der Auswahl des Vorschlags (P1) des ersten optischen Glases (V1) an einer Stelle des automatischen Rückstrahlerkopfes (T) anordnet (103);
- eine Auswahl des Vorschlags (P2) des zweiten optischen Glases (V2) empfängt (104);
- das zweite optische Glas (V2) beim Empfang der Auswahl des Vorschlags (P2) des zweiten optischen Glases (V2) an der Stelle des automatischen Rückstrahlerkopfes (T) anordnet (105);
- auf dem Auswahlbildschirm (ES) eine Vielzahl von Bestätigungssymbolen (S1, S2, S3) anzeigt (103, 105), die jeweils ein mögliches Ergebnis des Vergleichs angeben;
wobei das Verfahren (100) derart ist, dass:
- wenn das Steuermodul die Auswahl des Vorschlags (P1) des ersten optischen Glases (V1) zum ersten Mal empfängt, ist der Vorschlag (P2) des zweiten optischen Glases (V2) auf dem Auswahlbildschirm (ES) auswählbar und kein Bestätigungssymbol (S1, S2, S3) ist auf dem Auswahlbildschirm (ES) auswählbar;
- wenn das Steuermodul die Auswahl des Vorschlags (P2) des zweiten optischen Glases (V2) empfängt, ist der Vorschlag (P1) des ersten optischen Glases (V1) auf dem Auswahlbildschirm (ES) auswählbar und das erste und das zweite Bestätigungssymbol (S1, S2) sind auf dem Auswahlbildschirm (ES) auswählbar, wobei das erste Symbol (S1) angibt, dass das erste und das zweite optische Glas (V1, V2) gleich sind und wobei das zweite Symbol (S2) angibt, dass das zweite optische Glas (V2) besser ist als das erste optische Glas (V1);
- wenn das Steuermodul die Auswahl des Vorschlags (P1) des ersten optischen Glases (V1) zum zweiten Mal oder häufiger empfängt, ist der Vorschlag (P2) des zweiten optischen Glases (V2) auf dem Auswahlbildschirm (ES) auswählbar und das erste Symbol (S1) und ein drittes Symbol (S3) sind auf dem Auswahlbildschirm (ES) auswählbar, wobei das dritte Symbol (S3) angibt, dass das erste optische Glas (V1) besser ist als das zweite optische Glas (V2).

2. Verfahren (100) gemäß dem voranstehenden Anspruch, **dadurch gekennzeichnet, dass**, wenn der zweite Schritt (102) zum ersten Mal erfolgt ist, dieser von dem Steuermodul ohne Eingreifen eines Operators automatisch realisiert wird.

3. Verfahren (100) gemäß irgendeinem der voranstehenden Ansprüche, **dadurch gekennzeichnet, dass**, wenn das Steuermodul die Auswahl des Vorschlags (P1) des ersten optischen Glases (V1) zum ersten Mal empfängt, das Steuermodul kein Bestätigungssymbol (S1, S2, S3) auf dem Auswahlbildschirm (ES) anzeigt.

4. Verfahren (100) gemäß irgendeinem der voranstehenden Ansprüche, **dadurch gekennzeichnet, dass**, wenn das Steuermodul die Auswahl des Vorschlags (P2) des zweiten optischen Glases (V2) empfängt, das Steuermodul das erste und das zweite Bestätigungssymbol (S1, S2) anzeigt, das dritte Bestätigungssymbol (S3) aber nicht.

5. Verfahren (100) gemäß irgendeinem der voranstehenden Ansprüche, **dadurch gekennzeichnet, dass**, wenn das Steuermodul die Auswahl des Vorschlags (P1) des ersten optischen Glases (V1) zum zweiten Mal oder häufiger empfängt, das Steuermodul das erste und das dritte Bestätigungssymbol (S1, S3) anzeigt, das zweite Bestätigungsmodul (S2) aber nicht.

6. Steuermodul eines automatischen Rückstrahlerkopfes (T) zum Umsetzen eines Vergleichsverfahrens (100) des ersten und des zweiten optischen Glases gemäß irgendeinem der voranstehenden Ansprüche, wobei das Steuermodul aufweist:
- Mittel zum gleichzeitigen Anzeigen eines Vorschlags (P1) eines ersten optischen Glases (V1), eines Vorschlags (P2) eines zweiten optischen Glases (V2) und eine ganze oder teilweise Vielzahl von Bestätigungssymbolen (S1, S2, S3) auf einem Auswahlbildschirm (ES), die jeweils ein mögliches Ergebnis eines Vergleichs zwischen dem ersten und dem zweiten optischen Glas (V1, V2) angeben;
- Mittel zum Empfangen einer Auswahl des Vorschlages (P1, P2) des ersten oder des zweiten optischen Glases (V1, V2) oder eines Bestätigungssymboles (S1, S2, S3);
- Mittel zum Anordnen des genannten ersten oder des zweiten optischen Glases (V1, V2) an einer Stelle des automatischen Rückstrahlkopfes (T) beim Empfang der Auswahl des Vorschlags (P1, P2) des ersten oder des zweiten optischen Glases (V1, V2);
wobei die Anzeigemittel auf dem Auswahlbildschirm und die Empfangsmittel einer Auswahl derart sind, dass:
- wenn das Steuermodul die Auswahl des Vorschlags (P1) des ersten optischen Glases (V1) zum ersten Mal empfängt, ist der Vorschlag (P2) des zweiten optischen Glases (V2) auf dem Auswahlbildschirm (ES) auswählbar und kein Bestätigungssymbol (S1, S2 S3) ist auf dem Auswahlbildschirm auswählbar;
- wenn das Steuermodul die Auswahl des Vorschlags (P2) des zweiten optischen Glases (V2) empfängt, ist der Vorschlag (P1) des ersten optischen Glases (V1) auf dem Auswahlbildschirm (ES) auswählbar und das erste und das zweite Bestätigungssymbol (S1, S2) sind auf dem Auswählbildschirm (ES) auswählbar, wobei das erste Symbol (S1) angibt, dass das erste und das zweite optische Glas ((V1, V2) gleich sind und wobei das zweite Symbol (S2) angibt, dass das zweite optische Glas (V2) besser ist als das erste optische Glas (V1);
- wenn das Steuermodul die Auswahl des Vorschlags (P1) des ersten optischen Glases (V1) zum zweiten Mal oder häufiger empfängt, ist der Vorschlag (P2) des zweiten Glases (V2) auf dem Auswahlbildschirm (ES) auswählbar und das erste Symbol (S1) und ein drittes Symbol (S3) sind auf dem Auswahlbildschirm (ES) auswählbar, wobei das dritte Symbol (S3) angibt, dass das erste optische Glas (V1) besser ist als das zweite optische Glas (V2).

7. System zum Umsetzen eines Vergleichsverfahrens (100) des ersten und des zweiten optischen Glases gemäß irgendeinem der Ansprüche 1 bis 5, umfassend das Steuermodul eines automatischen Rückstrahlerkopfes (T) gemäß Anspruch 6 sowie den Auswahlbildschirm (ES).

8. System gemäß dem voranstehenden Anspruch, **dadurch gekennzeichnet, dass** es darüber hinaus einen Lesebildschirm umfasst.

9. System gemäß dem voranstehenden Anspruch, **dadurch gekennzeichnet, dass** der Auswahlbildschirm (ES) und der Lesebildschirm zwei unterschiedliche Bildschirme sind.

10. System gemäß Anspruch 8, **dadurch gekennzeichnet, dass** der Auswahlbildschirm (ES) und der Lesebildschirm ein und derselbe Bildschirm sind.

## Claims

1. Method (100) for comparing first and second optical lenses (V1, V2) by means of an automatic refractor head (T) controlled by a control module, the method (100) comprising the following steps according to which the control module:
- displays (101) on a selection screen (ES) a proposal (P1) for a first optical lens (V1) and a proposal (P2) for a second optical lens (V2);
- receives (102) a selection of the proposal (P1) for the first optical lens (V1);
- arranges (103) the first optical lens (V1) in a slot of the automatic refractor head (T) on reception of the selection of the proposal (P1) for the first optical lens (V1);
- receives (104) a selection of the proposal (P2) for the second optical lens (V2);
- arranges (105) the second optical lens (V2) in the slot of the automatic refractor head (T) on reception of the selection of the proposal (P2) for the second optical lens (V2);
- displays (103, 105) on the selection screen (ES) a plurality of validation symbols (S1, S2, S3) each indicating a possible result of the comparison;
the method (100) being such that:
- when the control module receives the selection of the proposal (P1) for the first optical lens (V1) for the first time, the proposal (P2) for the second optical lens (V2) is selectable on the selection screen (ES) and no validation symbol (S1, S2, S3) is selectable on the selection screen (ES);
- when the control module receives the selection of the proposal (P2) for the second optical lens (V2), the proposal (P1) for the first optical lens (V1) is selectable on the selection screen (ES) and first and second validation symbols (S1, S2) are selectable on the selection screen (ES), the first symbol (S1) indicating that the first and second optical lenses (V1, V2) are the same and the second symbol (S2) indicating that the second optical lens (V2) is better than the first optical lens (V1);
- when the control module receives the selection of the proposal (P1) for the first optical lens (V1) for the second time or more, the proposal (P2) for the second optical lens (V2) is selectable on the selection screen (ES) and the first symbol (S1) and a third symbol (S3) are selectable on the selection screen (ES), the third symbol (S3) indicating that the first optical lens (V1) is better than the second optical lens (V2).

2. Method (100) according to the preceding claim **characterised in that** when the second step (102) takes place for the first time, it is carried out automatically by the control module without intervention of an operator.

3. Method (100) according to any one of the preceding claims **characterised in that** when the control module receives the selection of the proposal (P1) for the first optical lens (V1) for the first time, the control module displays no validation symbol (S1, S2, S3) on the selection screen (ES).

4. Method (100) according to any one of the preceding claims **characterised in that** when the control module receives the selection of the proposal (P2) for the second optical lens (V2), the control module displays the first and second validation symbols (S1, S2) but not the third validation symbol (S3).

5. Method (100) according to any one of the preceding claims **characterised in that** when the control module receives the selection of the proposal (P1) for the first optical lens (V1) for the second time or more, the control module displays the first and third validation symbols (S1, S3) but not the second validation symbol (S2).

6. Module for controlling an automatic refractor head (T) for the implementation of a method (100) for comparing first and second optical lenses according to any one of the preceding claims, the control module having:
- means for displaying simultaneously on a selection screen (ES) a proposal (P1) for a first optical lens (V1), a proposal (P2) for a second optical lens (V2) and all or part of a plurality of validation symbols (S1, S2, S3) each indicating a possible result of a comparison between the first and second optical lenses (V1, V2);
- means for receiving a selection of the proposal (P1, P2) for the first or the second optical lens (V1, V2) or a validation symbol (S1, S2, S3);
- means for, on reception of the selection of the proposal (P1, P2) for the first or the second optical lens (V1, V2), arranging said first or second optical lens (V1, V2) in a slot of the automatic refractor head (T);
the means for displaying on the selection screen and the means for receiving a selection being such that:
- when the control module receives the selection of the proposal (P1) for the first optical lens (V1) for the first time, the proposal (P2) for the second optical lens (V2) is selectable on the selection screen (ES) and no validation symbol (S1, S2, S3) is selectable on the selection screen (ES);
- when the control module receives the selection of the proposal (P2) for the second optical lens (V2), the proposal (P1) for the first optical lens (V1) is selectable on the selection screen (ES) and first and second validation symbols (S1, S2) are selectable on the selection screen (ES), the first symbol (S1) indicating that the first and second optical lenses (V1, V2) are the same and the second symbol (S2) indicating that the second optical lens (V2) is better than the first optical lens (V1);
- when the control module receives the selection of the proposal (P1) for the first optical lens (V1) for the second time or more, the proposal (P2) for the second lens (V2) is selectable on the selection screen (ES) and the first symbol (S1) and a third symbol (S3) are selectable on the selection screen (ES), the third symbol (S3) indicating that the first optical lens (V1) is better than the second optical lens (V2).

7. System for the implementation of a method (100) for comparing first and second optical lenses according to any one of claims 1 to 5, comprising the module for controlling an automatic refractor head (T) according to claim 6 as well as the selection screen (ES).

8. System according to the preceding claim **characterised in that** it further comprises a reading screen.

9. System according to the preceding claim **characterised in that** the selection screen (ES) and the reading screen are two distinct screens.

10. System according to claim 8 **characterised in that** the selection screen (ES) and the reading screen are a single and same screen.
